# EUROPEAN PATENT APPLICATION

(11) **EP 2 036 981 A1**
(43) Date of publication of application: **18.03.2009**
(21) Application number: 07075801.6
(22) Date of filing: 12.09.2007
(51) Int. Cl.: C12N 15/11, A61K 51/04

(54) **Aptamers labeled with 18F**

(71) Applicant: Bayer Schering Pharma Aktiengesellschaft, 13353 Berlin (DE)
(72) Inventor: Friebe, Matthias, 13509 Berlin (DE); Dinkelburg, Ludger, 13464 Berlin (DE); Hecht, Maren, 10119 Berlin (DE); Dollé, Frédéric, 91940 Gometz-le Chatel (FR); Kuhnast, Bertrand, 92340 Bourg la Reine (FR); Hinnen, Francoise, 91430 Vauhallan (FR); Boisgard, Raphael, 91620 Nozay (FR); Tavitian, Bertrand, 75015 Paris (FR)

(57) **Abstract**

This invention relates to novel nucleotide-based compounds, methods of making radiolabeled compounds and use of such compounds for diagnostic imaging. Provided are compounds according to Formula (I)
A-B-L-[¹⁸F]c
wherein A stands for an aptamer, B is absent or stands for a bridging structure, L is a linker, and [¹⁸F]c is an 18F bearing chemical entity. Preferred embodiments of said compounds bind to Tenascin-C.

## Description

### FIELD OF THE INVENTION

This invention relates to novel nucleotide-based compounds, methods of making radiolabeled compounds and use of such compounds for diagnostic imaging.

### BACKGROUND OF THE INVENTION

### Tenascin-C as a potential target for diagnostic imaging in oncology

Besides other interesting targets, tenascin-C, a hexameric glycoprotein that is located primarily in the extracellular matrix (ECM) could serve as an appropriate target for specific molecular imaging of cancerous lesions. The protein is produced by epithelial cells and surrounding mesenchymal cells (see Harold P. Erickson, Annu. Rev. Cell Biol. 5 (1989) 71-92; R Chiquet-Ehrismann, EJ Mackie, CA Pearson, T Sakakura, Tenascin: an Extracellular Matrix Protein Involved in Tissue Interactions during Fetal Development and Oncogenesis, Cell 47 (1986) 131-139; R Chiquet-Ehrismann, P Kalla, CA Pearson, K Beck, M Chiquet, Tenascin Interferes with Fibronectin Action, Cell 53 (1988) 383-390). It is involved in cell proliferation, cancer invasion, metastasis and secondary tumor formation. It is also strongly present in embryonic development and in wound healing but its expression is only very limited in adult normal tissue (see e.g. Bourdon M.A., Wikstrand C.J., Furthermayr H., Metthews T.J., Bigner D.D., Cancer Res. 43 (1983) 2796-2805; N Hiraiwa, H Kida, T Sakakura, M Kusakabe, Induction of tenscin in cancer cells by interactions with embryonic mesenchyme mediated by a diffusible factor, Journal of Cell Science 104 (1993) 289-296). Tenascin-C is secreted by fibroblasts and glial cells in tissue culture. One of the experimental sources of human tenascin-C is the glioma cell line U251 MG, but also other cells such as U373, PC3 and F9 express large amounts of the protein. Each human tenascin-C subunit includes three types of structural modules: 14 1/2 epidermal growth factor-like repeats, 15 fibronectin (FN)type, three homology repeats, and a COOH-terminal knob which consists of 215 amino acids with homology to the globular domain of the β and γ chains of human fibrinogen.

Due to its high expression in a wide variety of tumors, the protein tenascin-C (TN-C) is an interesting target for multi tumor diagnosis and therapy (see AM Tókés, E Hortoványi, J Kulka, M Jäkel, T Kerény, A Kádár, Tenascin expression and angiogenisis in breast cancer, Pathol. Res. Pract. 195 (1999) 821-828; Y Soini, P Paakko, Teanscin immunoreactivity in lung tumors, Am. J. Clin. Path. 100 (1993) 145-150; S Riedl, A Faissner, P Schlag, A Aon-Herbay, K Koretz, P Möller, altered content and distribution of tenascin in colltis, coion adenoma and colorectal carcinoma, Gastroenterology 103 (1992) 400-406; MM Bonsanto, Klinische und funktionelle Aspekte der Tenascin-Expression im Zuge der Gliomprogression, http://www.ub.uni-heidelberg.de/archiv/4305). An antibody against TN-C has been generated and radiolabeled for testing its applicability in radiotherapy (see e.g. G Akabani, DA Reardon, RE Coleman, TZ Wong, SD Metzler, JE Bowsher, DP Barboriak, JM Provenzale, KL Greer, D DeJong, HS Friedman, AH Friedman, XG Zhao, CN Pegram, RE McLendon, DD Bigner, MR Zalutsky, Dosimetry and Radiographic Analysis of 131I-Labeled Anti-Tenascin 81 C6 Murine Monoclonal Antibody in Newly Diagnosed Patients with malignant Gliomas: A Phase II Study, The Journal of Nuclear Medicine 46 (2005) 1042-1050; G Paganelli, C Grana, M chinol, M Cremonesi, C De Cicco, F De Braud, C Robertson, S Zurrida, C Casadio, S Zoboli, AG Siccardi, U Veronesi, Antibody-guided three-step therapy for high grade glioma with yttrium-90, European Journal of Nuclear Medicine 26 (1999) 348-357; F Petronzelli, A Pelliccia, AM Anastasi, V D'Alessio, C Albertoni, A Rosi, B Leoni, C De Angelis, G Paganelli, G Palombo, M Dani, P Carminati, R De Santis, Improved Tumor Targeting by Combined Use of Two Antitenascin Antibodies, Clin Cancer Res 11 (2005) 7137s-7145s; MR Zalutsky, RP Moseley, HB Coakham, RE Coleman, DD Bigner, Pharmacokinetics and Tumor Localization of 131I-Labeled Anti-Tenascin Monoclonal Antibody 81 C6 in Patients with Gliomas and Other Intracranial Malignancies, Cancer Research 49 (1989) 2807-2813; G Akabani, I Cokgor, RE Coleman, DG Trotter, TZ Wong, HS Friedman, AH Friedman, A Garcia-Turner, JE Herndon II, D DeLong, RE McLendon, XG Zhao, CN Pegram, JM Provenzale, DD binger, MR Zalutsky, Dosimetry and Dose-Response Relationships in Newly Diagnosed Patients with Malignant Gliomas Treated with Iodine-131-Labeled Anti-Tenascin Monoclonal Antibody 81 C6 Therapy, Int. J. Radiation Oncology Biol. Phys. 46 (2000) 947- 958). A high tumor uptake of the radiolabeled antibody has been displayed in these studies. Despite the high affinity binding, this approach is fraught by the large size of antibodies which leads to a long blood-retention time and a slow tissue penetration. This in turn causes an unfavorable tumor-to-non-tumor distribution of the radiolabeled antibody. Furthermore, antibodies can be immunogenic and have high costs of production, two factors that limit their use in diagnostic applications. Synthetic oligonucleotides such as aptamers could overcome these problems. Aptamers are non-immunogenic and usually have a very selective binding to their target. There is hardly any interactions with other tissue which together with a mid-size molecular weight (7-20 kDa) leads to a fast body clearance. A potential binding site for aptamers could be the terminal knob of tenascin-C. Therefore, six possible binding sites would exist per tenascin-C molecule.

### Aptamers

Aptamers are synthetic DNA or RNA molecules that can be selected from oligonucleotide libraries based on their ability to bind a target molecule (M Famulok, G Mayer, Aptamers as Tools in Molecular Biology and Immunology, In: Combinatorial Chemistry in Biology, Current Topics in Microbiology and Immunology (M Famulok, CH Wong, EL Winnacker, Eds.), Springer Verlag Heidelberg, 1999, Vol. 243, 123-136). They fold into three dimensional structures by Watson-Crick base pairing. This three dimensional structure enables aptamers for molecular recognition and can lead to a high affinity and selectivity for a particular target. Their preshaped structures can minimize the entropic loss during binding to the target. Of high interest is their ability to bind to proteins with high specificity (L Cerchia, J Hamm, D Libri, B Tavitian, V de Franciscis, Nucleic acid aptamers in cancer medicine, FEBS Letters 528 (2002), 12-16). Binding affinities are usually in the sub-nanomolar range. It is noteworthy, that aptamers can selectively recognize minor sequence variations of biomolecules and even enantiomers of small molecules with usually several orders of magnitude (SM Nimjee, CP Rusconi, BA Sullenger, Aptamers: An Emerging Class of Therapeutics, Annu. Rev. Med. 56 (2005) 555-583; RR White, BA Sullenger, CP Rusconi, Developing aptamers into therapeutics, The Journal of Clinical Investigation 106 (2000) 929-934).

Aptamers binding to a variety of targets are described in the literature (REFs 62-65). Among those are aptamers with a high affinity for α-thrombin (MF Kubik, AW Stephens, D Schneider, RA Marlar, D Tasset, High-affinity RNA ligands to human α-thrombin, Nucleic Acids Research 22 (1994) 2619-2626), HIV-1 reverse transcriptase (C Tuerk, S MacDougal, L Gold, RNA pseudoknots that inhibit human immunodeficiency virus type 1 reverse transcriptase, Proc. Natl. Acad. Sci. U.S.A. 89 (1992) 6988-6992), and basic fibroblast growth factor (D Jellinek, CK Lynott, DB Rifkin, N Janjic, High-affinity RNA ligands to basic fibroblast growth factor inhibit receptor binding, Proc. Natl. Acad. Sci. U.S.A. 90 (1993) 11227-11231). The first aptamer drug, that gained approval by the FDA is Macugen, a molecule that was identified by NeXstar (NX-1838) (I Melnikova, Wet age-related macular degeneration, Nature Reviews 4 (2005) 711-712). It specifically targets the vascular endothelial growth factor (VEGF), a protein promoting blood vessel growth, which is a hallmark of neovascular (wet) macular degeneration. Hence, it slows the progress of wet macular degeneration, the leading cause of blindness in people older than 50. Due to their high selectivity for a specific target, aptamers have a high potential as targeting probes in radiodiagnostic imaging and therapy. Their favorable size, which lies in-between that of antibodies and peptides, combined with the high potential binding affinity may allow for high signal to noise ratios.

Such high affinity binding aptamers can be generated by an *in vitro* process called SELEX (Systematic Evolution of Ligands by EXponential enrichment). This iterative process was established by Gold et al. in 1990 (JM Bacher AD Elligngton, Nucleic acid selection as a tool for drug discovery, DDT 3 (1998) 265- 273; L Gold, B Polisky, O Uhlenbeck, M Yarus, Diversity of Oligonucleotide Functions, Annu. Rev. Biochem. 64 (1995) 763-797) based on the selection and amplification of the anticipated high affinity binding aptamer. The starting library for the selection of oligonucleotide aptamers contains single stranded DNA or RNA oligonucleotides with a central region of randomized sequences (up to 10¹⁵ different sequences) abutted on constant regions for subsequent reverse transcription and amplification. These single stranded oligonucleotides are incubated with a target molecule (e.g. tenascin-C) and then partitioned by passage through a nitrocellulose filter: Those oligonucleotides bound to the protein are collected and reverse transcribed into RNA (or DNA), and amplified with the polymerase chain reaction (PCR) to create a new pool that can be used for the next round of SELEX. After several repetitions (usually 8-12), typically performed with increasing stringency, the selected ligands are sequenced and evaluated for their binding capabilities.

### TTA 1: A Tenascin-C binding aptamer

An aptamer against the human matrix protein tenascin-C has been identified via the SELEX process using a tenascin-C expressing U251 cell line as well as purified human tenascin-C protein and was first published by Hicke et al (BJ Hicke, C Marion, YF Chang, T Gould, CK Lynott, D Parma, PG Schmidt, S Warren, Tenascin-C Apamers Are Generated Using Tumor Cells and Purified Protein, The Journal of Biological Chemistry 276 (2001) 48644 - 48654; WO 01/09390 A1). Random 2'F stabilized pyrimidine RNA sequences were incubated with the protein expressing cell line. After 9 rounds the best binding sequences were incubated with the purified tenascin-C. Two more rounds were needed to discover a sequence with a binding affinity to tenascin-C of 1 x 10⁻⁹ M. The final aptamer was then modified to be able to withstand *in vivo* degradation. The modifications covered size minimization, further nuclease stabilization and the attachment of a bioconjugation handle. The size minimization included the replacement of 17 bases by a (CH₂O)₆ group without loss in affinity. Nuclease resistance was already introduced before the SELEX process by using 2'F pyrimidines, further stabilization was achieved by converting 2'OH purines into 2'OMe purines. Hicke et al. found that the conversion of 2'OH in five guanodines (G) led to a loss of activity: G¹, G⁹, G¹¹, G¹⁴ and G¹⁷. Hence, these five nucleotides remained 2'OH. In addition a 3'cap was added to further increase the nuclease stability. Finally, a C₆-NH₂ linker was added to the 5' end of the oligonucleotide and the final aptamer was tested again for its binding affinity. The synthetic oligonucleotide has a K_{D} of 5 x 10⁻⁹ M which represents a five fold reduction in affinity from the parent full size aptamer. The final molecule was named TTA1, which stands for Tenascin-C targeting aptamer-1 and the sequence was determined to be:

NH2-C6-5'-GGG AGG ACG-(CH2CH2O)6-CGU CGC CGU AAU GGA UGU UUU GCU CCC
UG-3'-3'-T

In conclusion, TTA1 consists of 39 nucleotides which are stabilized against nuclease degradation by the substitution of the 2'OH group versus a 2'fluoro substituent of the ribose backbone of pyrimidine and versus 2'-O-methyl substituents in the purine nucleic acids. In addition, the 3' end was protected against exonuclease degradation by inverting the 3' nucleotide to form a new 5'OH, with a 3'-3' linkage to a penultimate base (REF 128). The amine group on the linker can be used for the introduction of e.g. chelating ligands or dyes. TTA1 is characterized by a 13.3 kDa molecular weight and a binding affinity of K_{D} = 5 nM for human tenascin-C.

TTA1 coupled to mercapto acetyl diglycine (MAG₂), an N₃S coordinating ligand, suitable for the subsequent radio labeling with [^{99m}Tc(V)O]³⁺, is the current lead structure. It is assumed that stem 2 and stem 3 are vital for the binding to the target protein while stem 1 is important for the structural stability (REF 128).

The radiolabeled lead compound was tested *in vitro* and *in vivo.* An organ distribution study in tumor bearing mice revealed a significant tumor uptake of the lead compound along with a considerable uptake of activity in the excretory organs such as liver and kidneys. A slow hepatobilliary clearance concomitant with a significant intestinal uptake usually leads to unfavorable diagnostic imaging due to a rather high background activity.

The object of the present invention is to provide new compounds, that have improved properties and are suitable for diagnostic imaging.

### SUMMARY OF THE INVENTION

The present invention provides novel compounds of Formula I that are labeled with ¹⁸F.

The present invention also provides compositions comprising compound of Formula I and pharmaceutically acceptable salts of inorganic or organic acids, hydrates, complexes, esters, amides, solvates and prodrugs thereof..

The invention further provides a method of imaging diseases, the method comprising introducing into a patient a detectable quantity of a labeled compound of Formula I or to pharmaceutically acceptable salts of inorganic or organic acids, hydrates, complexes, esters, amides, solvates and prodrugs thereof and detecting an image.

The invention provides the compounds of Formula I for use as a medicament.

Another aspect of the invention is directed to the use of compounds of Formula I for the manufacture of a medicament.

Another aspect of the invention is directed to the use of labeled compounds of Formula I for PET imaging.

More specifically the compounds of this invention are useful for the imaging of a variety of cancers including but not limited to: carcinoma such as bladder, breast, colon, kidney, liver, lung, including small cell lung cancer, esophagus, gall-bladder, ovary, pancreas, stomach, cervix, thyroid, prostate, and skin, hematopoetic tumors of lymphoid and myeloid lineage, tumors of mesenchymal origin, tumors of central peripheral nervous systems, other tumors, including melanoma, seminoma, teratocarcinoma, osteosarcoma, xeroderma pigmentosum, keratoxanthoma, thyroid follicular cancer, and Karposi's sarcoma.

### DETAILED DESCRIPTION OF THE INVENTION

In a **first aspect,** the present invention is directed to compounds according to Formula I:

**A-B-L- [¹⁸F]c** **(I)**

wherein,
**A** stands for an aptamer,
**B** is absent or stands for a bridging structure,
**[¹⁸F]c** is an 18F bearing chemical entity, and
**L** is a linker.

For the purpose of this invention, the term "aptamer" refers to synthetic molecules, comprising from 4 to 100 nucleotides, wherein at least two single nucleotides are connected to each other via a phosphodiester linkage. Said aptamers have the ability to bind specifically to a target molecule (see e.g. M Famulok, G Mayer, Aptamers as Tools in Molecular Biology and Immunology, In Combinatorial Chemistry in Biology, Current Topics in Microbiology and Immunology (M Famulok, CH Wong, EL Winnacker, Eds.), Springer Verlag Heidelberg, 1999, Vol. 243, 123-136). There are many ways known to the skilled person of how to generate such aptamers that have specificity for a certain target molecule. An example is given in WO 2001/09390 table 3 page 527-529, the disclosure of which is hereby incorporated by reference. Said aptamers may comprise substituted or non-substituted natural and non-natural nucleotides. Aptamers can be synthesized in vitro using e.g. an automated synthesizer (see e.g. S Verma, F Eckstein, Modified Oligonucleotides: Synthesis and Strategy for Users, Annual Reviews of Biochemistry 67 (1998) 99-134). Aptamers according to the present invention can be stabilized against nuclease degradation e.g. by the substitution of the 2'-OH group versus a 2'-fluoro substituent of the ribose backbone of pyrimidine and versus 2'-O-methyl substituents in the purine nucleic acids. In addition, the 3' end of an aptamer can be protected against exonuclease degradation by inverting the 3' nucleotide to form a new 5'-OH group, with a 3' to 3' linkage to a penultimate base (see e.g. BJ Hicke, C Marion, YF Chang, T Gould, CK Lynott, D Parma, PG Schmidt, S Warren, Tenascin-C Aptamers Are Generated Using Tumor Cells and Purified Protein, The Journal of Biological Chemistry 276 (2001) 48644 - 48654)

For the purpose of this invention, the term "nucleotide" refers to molecules comprising a nitrogen-containing base, a 5-carbon sugar, and one or more phosphate groups. Examples of said base comprise, but are not limited to, adenine, guanine, cytosine, uracil, and thymine. Also non-natural, substituted or non-substituted bases are included. Examples of 5-carbon sugar comprise, but are not limited to, D-ribose, and D-2-desoxyribose. Also other natural and non-natural, substituted or non-substituted 5-carbon sugars are included. Nucleotides as used in this invention may comprise from one to three phosphates.

In preferred embodiments of compounds according to Formula I, aptamers **A** are selected from Tenascin-C binding aptamers.
For the purpose of this invention, the term "Tenascin-C binding" shall have the following meaning: Aptamers that are Tenascin-C binding show a binding affinity towards Tenascin-C of 1 mM or less, wherein less means a more specific affinity in the nanomolar an/or subnanomolar range. This binding affinity is measured using a well established assay, which is easy to perform and well known to the person skilled in the art. This assay is described in detail e.g. in WO 2001/09390 example 4 page 22, which is hereby incorporated by reference.

Further preferred embodiments of said aptamers **A** have a binding affinity towards Tenascin-C of 100nM or less, with a further preferred range of from 50nM to 1 pM.

In preferred embodiments of compounds according to Formula I, aptamers **A** comprise from 10 to 50 nucleotides.

In preferred embodiments of compounds according to Formula I, aptamers A are selected from the group of

TTA4: 5'-mGmGmG mAmGG mAfCmG-(CH2CH2O)6-fCGfU fCGfC fCGfU mAmAfU
mGmGmA fUmGfU fUfUfU mGfCfU fC(C6-NH2)CfC-3'-3'-T ,

**TTA5:** 5'-(aminomodifier C2 dT)-mA mGmGmG mAmGG mAfCmG-(CH2CH2O)6-fCGfU
fCGfC fCGfU mAmAfU mGmGmA fUmGfU fUfUfU mGfCfU fCfCfC fUmG-3'-3'-T ,

**TTA7:** 5'-LGLGLG LALGmG mAfCrG-(CH2OCH2)6-fCGfU fCGfC fCGfU mAmAfU mGmGmA
fUmGfU fUfUfU mGLCLU LCLCLC-(CH2)6NH2-3' , and

TTA8: 5'-mGmGmG mAmGmG mAfCrG-(CH2OCH2)6-fCGfU fCGfC fCGfU mAmAfU
mGmGmA fUmGfU fUfUfU mGmCmU mCmCmC-(CH2)6NH2-3'.

The 5' end of an aptamer may be substituted at the free phosphate group with a bridging structure that allows for the conjugation of further chemical structures, e.g. the linker L. This bridging structure comprises a substituted or un-substituted lower un-branched or branched alkyl, lower un-branched or branched alkene, or substituted or un-substituted lower un-branched or branched aldehyde carrying an amine functionality. As used in the specification and appended claims, unless specified to the contrary, the term "lower un-branched or branched alkyl" shall have the following meaning: a straight or branched chain monovalent or divalent radical consisting solely of carbon and hydrogen, containing no unsaturation and having from one to eight carbon atoms, e.g. but not limited to methyl, ethyl, n-propyl, n-pentyl, 1,1-dimethylethyl (t-butyl), n-heptyl, and the like. As used in the specification and appended claims, unless specified to the contrary, the term "lower un-branched or branched alkene" shall have the following meaning: a straight or branched chain monovalent or divalent radical consisting solely of carbon and hydrogen, containing at least one unsaturation and having from one to eight carbon atoms. As used in the specification and appended claims, unless specified to the contrary, the term "lower un-branched or branched aldehyde" shall have the following meaning: a straight or branched chain monovalent or divalent radical consisting solely of carbon and hydrogen and at least one aldehyde group and having from one to eight carbon atoms.

In preferred embodiments of compounds according to Formula I, the bridging structure **B** is hexyl amine.

In further preferred embodiments of compounds according to Formula I, aptamer **A** including the bridging structure **B,** the **A-B** portion of Formula I, is selected from the group of

TTA1: 5'-NH2-C6-O-PO2-O-GGG AGG ACG-(CH2CH2O)6-CGU CGC CGU AAU GGA UGU
UUU GCU CCC UG-3'-3'-T ,

**TTA2:** 5'-NH2-C6-O-PO2-O-mGmGmG mAmGG mAfCmG-(CH2CH2O)6-fCGfU fCGfC fCGfU
mAmAfU mGmGmA fUmGfU fUfUfU mGfCfU fCfCfC UmG-3'-3'-T ,

**TTA3:** 5'-NH2-C6-O-PO2-O-mGmGmG mAmGG mAfCmG-(CH2CH2O)6-fCGfU fCGfC fCGfU
mAmAfU mGmGmA fUmGfU fUfUfU mGfCfU fCfCfC UG-3'-3'-T ,

TTA6: 5'-NH2-C6-O-SPO-O-mGmGmG mAmGG mAfCmG-(CH2CH2O)6-fCGfU fCGfC fCGfU
mAmAfU mGmGmA fUmGfU fUfUfU mGfCfU fCfCfC fUmG-3'-3'-T .

For the purpose of this invention, the term "linker" refers to a chemical entity that connects the aptamer A via an amine group of the bridging structure B or of the aptamer with the group [¹⁸F]c. The linker itself can be a bond or is characterized by the presence of a free mercapto (SH)-functionality that can be used to connect the group [¹⁸F]c to the molecule. Examples of such linker L are, but are not limited to, a bond, substituted or un-substituted lower un-branched or branched alkyl, lower un-branched or branched alkene, or lower un-branched or branched aldehyde carrying at least one mercapto-group, a chemical structure carrying at least one free mercapto-group comprising one amino acid or an amino acid sequence of two (2) to twenty (20) amino acids, or any combinations thereof.

In preferred embodiments of compounds according to Formula I, **L** is a chemical structure carrying at least one free mercapto-group, wherein said chemical structure comprises an amino acid sequence of two (2) to ten (10) amino acids, a mercaptoacetyl-glycine-glycine group MAG₂ (see e.g. S Hilger, MC Willis, M Wolters, WA Pieken, Tc-99m-labeling of modified RNA, Nucleosides Nucleotides 18 (1999) 1479 - 1481, B Johannsen, H Spies, Technetium(V) Chemistry as Relevant to Nuclear Medicine, Topics in Current Chemistry 176 (1996) 78 - 120), a mercaptobutyrimidyl group, a mercapto-substituted C₁-C₄ alkyl group, a mercapto-substituted C₁-C₄ alkene group, or a mercapto-substituted C₁-C₄ aldehyde group.

For the purpose of the present invention the term "amino acid sequence" is defined herein as a polyamide obtainable by polycondensation of at least two (2) amino acids. For the purpose of the present invention the term "amino acid" means any molecule comprising at least one amino group and at least one carboxyl group, but no peptide bond within the molecule. In other words, an amino acid is a molecule that has a carboxylic acid functionality and an amine nitrogen having at least one free hydrogen, preferably in alpha position thereto, but no amide bond in the molecule structure. Thus, a dipeptide having a free amino group at the N-terminus and a free carboxyl group at the C-terminus is not to be considered as a single "amino acid" in the above definition. An amide bond as used herein means any covalent bond having the structure 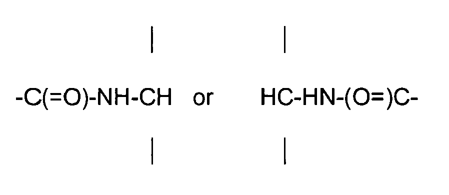 wherein the carbonyl group is provided by one molecule and the NH-group is provided by the other molecule to be joined. The amide bonds between two (2) adjacent amino acid residues which are obtained from such a polycondensation are defined as "peptide bonds". Optionally, the nitrogen atoms of the polyamide backbone (indicated as NH above) may be independently alkylated, e.g. with -C₁-C₆-alkyl, preferably -CH₃.

For the purpose of the specification an amino acid residue is derived from the corresponding amino acid by forming a peptide bond with another amino acid.

For the purpose of the specification an amino acid sequence may comprise naturally occurring and/or artificial amino acid residues, proteinogenic and/or non-proteinogenic amino acid residues. The non-proteinogenic amino acid residues may be further classified as (a) homo analogues of proteinogenic amino acids, (b) β-homo analogues of proteinogenic amino acid residues and (c) further non-proteinogenic amino acid residues.

Accordingly, the amino acid residues are derived from the corresponding amino acids, e.g. from
- proteinogenic amino acids, namely Ala, Arg, Asn, Asp, Cys, Gln, Glu, Gly, His, Ile, Leu, Lys, Met, Phe, Pro, Ser, Thr, Trp, Tyr and Val; or
- non-proteinogenic amino acids, such as
   o homo analogues of proteinogenic amino acids wherein the sidechain has been extended by a methylene group, e.g. Homoalanine (Hal), Homoarginine (Har), Homocysteine (Hcy), Homoglutamine (Hgl), Homohistidine (Hhi), Homoisoleucine (Hil), Homoleucine (Hle), Homolysine (Hly), Homomethionine (Hme), Homophenylalanine (Hph), Homoproline (Hpr), Homoserine (Hse), Homothreonine (Hth), Homotryptophane (Htr), Homotyrosine (Hty) and Homovaline (Hva);
   o β-homo analogues of proteinogenic amino acids wherein a methylene group has been inserted between the α-carbon and the carboxyl group yielding β-amino acids, e.g. β-Homoalanine (βHal), β-Homoarginine (βHar), β-Homoasparagine (βHas), β-Homocysteine (βHcy), β-Homoglutamine (βHgl), β-Homohistidine (βHhi), β-Homoisoleucine (βHil), β-Homoleucine (βHle), β-Homolysine (βHly), β-Homomethionine (βHme), β-Homophenylalanine (βHph), β-Homoproline (βHpr), β-Homoserine (βHse), β-Homothreonine (βHth), β-Homotryptophane (βHtr), β-Homotyrosine (βHty) and β-Homovaline (βHva);
   o further non-proteinogenic amino acids, e.g. α-Aminoadipic acid (Aad), β-Aminoadipic acid (βAad), α-Aminobutyric acid (Abu), α-Aminoisobutyric acid (Aib), β-Alanine (βAla), 4-Aminobutyric acid (4-Abu), 5-Aminovaleric acid (5-Ava), 6-Aminohexanoic acid (6-Ahx), 8-Aminooctanoic acid (8-Aoc), 9-Aminononanoic acid (9-Anc), 10-Aminodecanoic acid (10-Adc), 12-Aminododecanoic acid (12-Ado), α-Aminosuberic acid (Asu), Azetidine-2-carboxylic acid (Aze), β-Cyclohexylalanine (Cha), Citrulline (Cit), Dehydroalanine (Dha), γ-Carboxyglutamic acid (Gla), α-Cyclohexylglycine (Chg), Propargylglycine (Pra), Pyroglutamic acid (Glp), α-tert-Butylglycine (Tle), 4-Benzoylphenylalanine (Bpa), δ-Hydroxylysine (Hyl), 4-Hydroxyproline (Hyp), allo-Isoleucine (alle), Lanthionine (Lan), (1-naphthyl)alanine (1-Nal), (2-naphthyl)alanine (2-Nal), Norleucine (Nle), Norvaline (Nva), Ornithine (Orn), Phenylglycin (Phg), Pipecolic acid (Pip), Sarcosine (Sar), Selenocysteine (Sec), Statine (Sta), □-Thienylalanine (Thi), 1,2,3,4-Tetrahydroisochinoline-3-carboxylic acid (Tic), allo-Threonine (aThr), Thiazolidine-4-carboxylic acid (Thz), γ-Aminobutyric acid (GABA), iso-Cysteine (iso-Cys), Diaminopropionic acid (Dpr), 2,4-Diaminobutyric acid (Dab), 3,4-Diaminobutyric acid (γ,βDab), Biphenylalanine (Bip), Phenylalanine substituted in para-position with -C₁-C₆-alkyl, -halide, -NH₂ or -CO₂H (Phe(4-R) wherein R = -C₁-C₆-alkyl, -halide, -NH₂, or -CO₂H); peptide nucleic acids (PNA, cf. P.E. Nielsen, Acc.Chem.Res. 32, 624-30)
- or their N-alkylated analogues, such as their N-methylated analogues.

Cyclic amino acids may be proteinogenic or non-proteinogenic, such as Pro, Aze, Glp, Hyp, Pip, Tic and Thz.

For further examples and details reference can be made to e.g. J.H. Jones, J. Peptide Sci. 2003, 9, 1-8 which is incorporated herein by reference.

The terms "non-proteinogenic amino acid" and "non-proteinogenic amino acid residue" also encompasses derivatives of proteinogenic amino acids. For example, the sidechain of a proteinogenic amino acid residue may be derivatized thereby rendering the proteinogenic amino acid residue "non-proteinogenic". The same applies to derivatives of the C-terminus and/or the N-terminus of a proteinogenic amino acid residue terminating the amino acid sequence.

For the purpose of the specification a proteinogenic amino acid residue is derived from a proteinogenic amino acid selected from the group consisting of Ala, Arg, Asn, Asp, Cys, Gln, Glu, Gly, His, Ile, Leu, Lys, Met, Phe, Pro, Ser, Thr, Trp, Tyr and Val either in L- or D-configuration; the second chiral center in Thr and Ile may have either R- or S-configuration. Therefore, for example, any posttranslational modification of an amino acid sequence, such as N-alkylation, which might naturally occur renders the corresponding modified amino acid residue "non-proteinogenic", although in nature said amino acid residue is incorporated in a protein.

In preferred embodiments of compounds according to Formula I, **[¹⁸F]c** is:

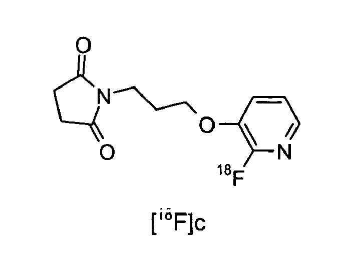

In the following Schemes 1 and 2, a general route of synthesis is outlined that can be used to produce compounds over the whole breadth of Formula I. Suitable reaction conditions are described e.g. in WO 2004/002984, and in de Bruin et al. "Design, Synthesis and Radiosynthesis of a New [18F]Fluoropyridine-Based Maleimide Reagent for the Labeling of Peptides and Proteins", Bioconj. Chem. 2005, 16(2), 406-420, the content of which is incorporated by reference.

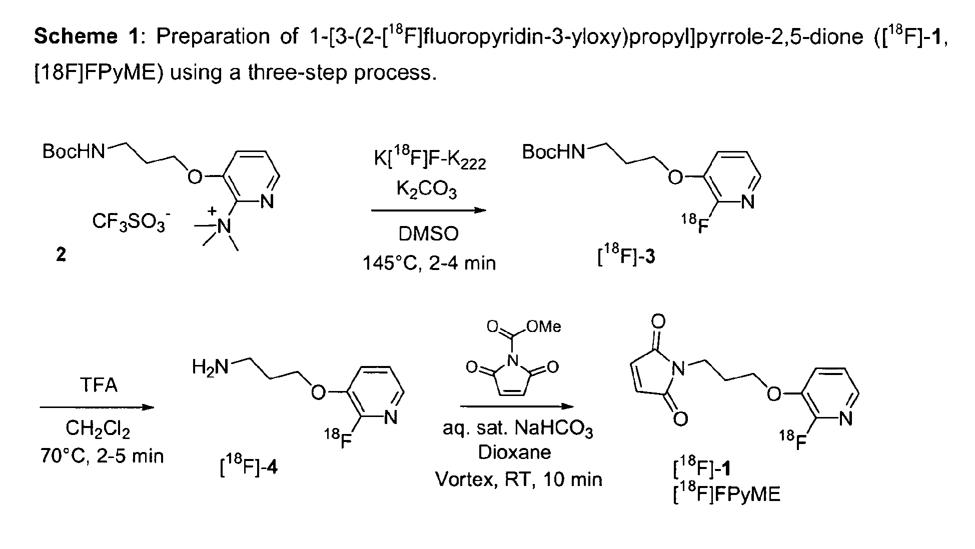

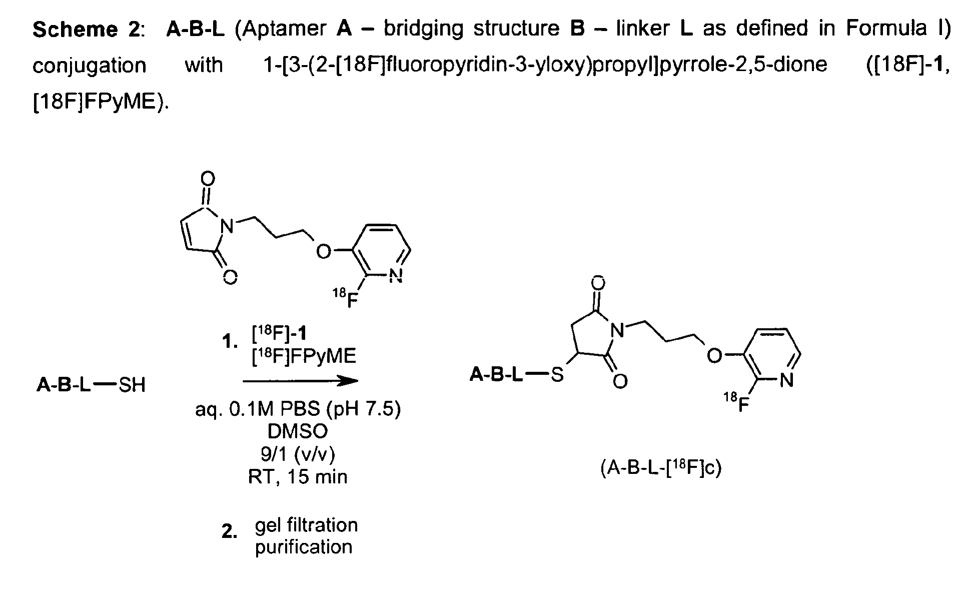

Unless otherwise specified, when referring, to the compounds of Formula I per se, as well as to any pharmaceutical composition thereof comprising a compound according to Formula I the present invention includes all of the hydrates, solvates, complexes, and prodrugs of the compounds of the invention. Prodrugs are any covalently bonded compounds, which releases the active parent pharmaceutical according to Formula I.
The term "prodrug"as used throughout this text means the pharmacologically acceptable derivatives such as esters, amides and phosphates, such that the resulting in vivo biotransformation product of the derivative is the active drug as defined in the compounds of formula (I). The reference by Goodman and Gilman (The Pharmaco- logical Basis of Therapeutics, 8 ed, McGraw-HiM, Int. Ed. 1992,"Biotransformation of Drugs", p 13-15) describing prodrugs generally is hereby incorporated. Prodrugs of a compound of the present invention are prepared by modifying functional groups present in the compound in such a way that the modifications are cleaved, either in routine manipulation or in vivo, to the parent compound. Prodrugs of the compounds of the present invention include those compounds wherein for instance a hydroxy group, such as the hydroxy group on the asymmetric carbon atom, or an amino group is bonded to any group that, when the prodrug is administered to a patient, cleaves to form a free hydroxyl or free amino, respectively.

Typical examples of prodrugs are described for instance in WO 99/33795, WO 99/33815, WO 99/33793 and WO 99/33792 all incorporated herein by reference.
Prodrugs are characterized by excellent aqueous solubility, increased bioavailability and are readily metabolized into the active inhibitors in vivo.

If a chiral center or another form of an isomeric center is present in a compound according to Formula I of the present invention, all forms of such isomer, including enantiomers and diastereoisomers, are intended to be covered herein. Compounds containing a chiral center may be used as racemic mixture or as an enantiomerically enriched mixture or the racemic mixture may be separated using well-known techniques and an individual enantiomer maybe used alone. In case in which compounds have unsaturated carbon-carbon bonds double bonds, both the cis-isomer and trans-isomers are within the scope of this invention. In cases wherein compounds may exist in tautomeric forms, such as keto-enol tautomers, each tautomeric form is contemplated as being included within this invention whether existing in equilibrium or predominantly in one form.

The invention also provides compositions comprising compound of Formula I and a pharmaceutically carrier, diluent, excipient or adjuvant may include any and all solvents, dispersion media, antibacterial and antifungal agents, isotonic agents, enzyme inhibitors, transfer ligands such as glucoheptonate, tartrate, citrate, or mannitol, and the like. Suitable pharmaceutical acceptable carriers are known to the person skilled in the art. In this regard reference can be made to *e.g.*, Remington's Practice of Pharmacy, 11th ed. and in J. of. Pharmaceutical Science & Technology, Vol. 52, No. 5, Sept-Oct., p. 238-311 see table page 240 to 311, both publication include herein by reference. Such compositions may be formulated as sterile, pyrogen-free, parenterally acceptable aqueous solution which may optionally be supplied in lyophilized form. The compositions of the invention may be provided as components of kits which may include buffers, additional vials, instructions for use, and the like.

In a **second aspect** of the invention, compounds according to Formula I are provided for use as medicament or pharmaceutical.
The compounds according to Formula I are provided for the manufacture of a medicament or pharmaceutical.
Preferably the medicament or pharmaceutical is for the treatment of cancer.
More preferably, cancers including but not limited to: carcinoma such as bladder, breast, colon, kidney, liver, lung, including small cell lung cancer, esophagus, gall-bladder, ovary, pancreas, stomach, cervix, thyroid, prostate, and skin, hematopoetic tumors of lymphoid and myeloid lineage, tumors of mesenchymal origin, tumors of central peripheral nervous systems, other tumors, including melanoma, seminoma, teratocarcinoma, osteosarcoma, xeroderma pigmentosum, keratoxanthoma, thyroid follicular cancer, and Karposi's sarcoma.

The present invention is also directed to a method for treatment comprising the step of introducing into a patient a suitable quantity of a labeled compound of Formula I.

In a **third aspect** of the invention, compounds according to Formula I are provided for use as diagnostic imaging agent, preferably as imaging agent for PET applications.
The compounds according to Formula I are provided for the manufacture of a diagnostic imaging agent, more preferably a PET imaging agent.
Preferably the diagnostic imaging agent or imaging agent is for the imaging cancer.
More preferably, cancers including but not limited to: carcinoma such as bladder, breast, colon, kidney, liver, lung, including small cell lung cancer, esophagus, gall-bladder, ovary, pancreas, stomach, cervix, thyroid, prostate, and skin, hematopoetic tumors of lymphoid and myeloid lineage, tumors of mesenchymal origin, tumors of central peripheral nervous systems, other tumors, including melanoma, seminoma, teratocarcinoma, osteosarcoma, xeroderma pigmentosum, keratoxanthoma, thyroid follicular cancer, and Karposi's sarcoma.

The present invention is also directed to a method for imaging comprising the step of introducing into a patient a suitable quantity of a labeled compound of Formula I and detecting an image of a tissue or patient.

In a **fourth aspect** of the invention, compound according to Formula I are useful for quantify the in vivo level of expression of Tenascin-C.

The radioactively labeled compounds according to Formula I provided by the invention may be administered intravenously in any pharmaceutically acceptable carrier, e.g. conventional medium such as an aqueous saline medium, or in blood plasma medium, as a pharmaceutical composition for intravenous injection. Such medium may also contain conventional pharmaceutical materials such as, for example, pharmaceutically acceptable salts to adjust the osmotic pressure, buffers, preservatives and the like. Among the preferred media are normal saline and plasma. Suitable pharmaceutical acceptable carriers are known to the person skilled in the art. In this regard reference can be made to e.g. Remington's Practice of Pharmacy, 11th ed.

The concentration of the compound according to Formula I and the pharmaceutically acceptable carrier, for example, in an aqueous medium, varies with the particular field of use. A sufficient amount is present in the pharmaceutically acceptable carrier when satisfactory visualization of the imaging target (e.g. a tumor) is achievable.
In accordance with the invention, the radiolabeled compounds according to Formula I either as a neutral complex or as a salt with a pharmaceutically acceptable counter-ion are administered in a single unit injectable dose. Any of the common carriers known to those with skill in the art, such as sterile saline solution or plasma, can be utilized after radiolabeling for preparing the injectable solution to diagnostically image various organs, tumors and the like in accordance with the invention. Generally, the unit dose to be administered for a diagnostic agent has a radioactivity of about 0.1 mCi to about 100 mCi, preferably 1 mCi to 20 mCi. For a radiotherapeutic agent, the radioactivity of the therapeutic unit dose is about 10 mCi to 700 mCi, preferably 50 mCi to 400 mCi. The solution to be injected at unit dosage is from about 0.01 ml to about 30 ml. For diagnostic purposes after intravenous administration, imaging of the organ or tumor in vivo can take place in a matter of a few minutes. However, imaging takes place, if desired, in hours or even longer, after injecting into patients. In most instances, a sufficient amount of the administered dose will accumulate in the area to be imaged within about 0.1 of an hour to permit the taking of scintigraphic images. Any conventional method of scintigraphic imaging for diagnostic purposes can be utilized in accordance with this invention.

### BRIEF DESCRIPTION OF THE FIGURES

**Figure 1****:**
   Representative PET-scan images of a rat at successive times after injection of [18F]TTA1. Times indicated are in minutes after injection.
**Figure 2**
   Time activity curves of radioactivity concentrations in rats after iv injection of [18F]TTA1. Mean plus SD of 4 animals of the radioactivity concentration in the organs drawn from the PET images.
**Figure 3**
   Biodistribution from ex vivo organ counting at 60, 90 and 270 minutes after iv injection of [18F]TTA1 to mice bearing U251 tumour xenografts. Mean + SD of 4 animals.
**Figure 4**
   Typical PET images after injection of [18F]TTA1 to mice bearing U251 tumour xenografts. Upper panel : projection images. Lower panel: coronai sections. Colour scaies are the same for all images inside each panel.
**Figure 5**
   Time activity curves of radioactivity concentrations in mice bearing U251 tumours after iv injection of [18F]TTA1. Mean plus SD of 4 animals of the radioactivity concentrations in the organs drawn from the PET images.
**Figure 6**
   Tumour to muscle ratio of radioactivity concentrations in mice bearing U251 tumours after iv injection of [18F]TTA1

### EXAMPLES

### Example 1: Preparation of [18F]c-TTA1

**Chemicals.** Chemicals were purchased from standard commercial sources (Aldrich, Fluka or Sigma France) and were used without further purification, unless stated otherwise. Oxygen-18-enriched water ([¹⁸O]H₂O, > 95%) was purchased from CortecNet (Paris, France).

**Analytical Methods.** Thin layer chromatographies (TLCs) were run on pre-coated plates of silica gel 60F₂₅₄ (Merck). The compounds were localized (1) when possible at 254 nm using a UV-lamp and/or (2) by dipping the TLC-plates in a 1 % ethanolic ninhydrin solution and heating on a hot plate. Radioactive spots were detected using a Berthold TraceMaster 20 automatic TLC linear analyser. High pressure liquid chromatographies (HPLCs) were performed on Waters or Shimadzu systems: [HPLC A]: Equipment: a Waters 510 pump, a Shimadzu SPD10-AVP UV-multi-wavelength spectrometer and a Geiger-Müller detector; column: semipreparative SiO₂ Zorbax^{®} Rx-SIL, Hewlett Packard (250 x 9.4 mm; porosity: 5 µm); solvents and conditions: isocratic elution with heptane/EtOAc: 60/40 (v/v); flow rate: 5 mL/min; temperature: RT; absorbance detection at □ = 254 nm. [HPLC B]: Equipment: a Waters 600 Pump, a Waters 600 Gradient Controller, a Hewlett Packard Series 1100 multi-wavelength spectrometer and a Flow One Scintillation Analyzer Packard equipped with a positron dedicated cell for radioactivity monitoring ; column: semi preparative C18, µBondapak^{™}, Waters (300 x 7.8 mm; porosity: 10 □m); solvents and conditions: triethylammonium acetate, 50 mM, pH 7 (TEAA) and acetonitrile; solvents and conditions: triethylammonium acetate, 50 mM, pH 7 (TEAA) and acetonitrile; gradient elution: linear 5 min from 95/5 to 90/10 (TEAA/acetonitrile, v:v) then linear 10 min from 90/10 to 70/30 (v:v) and wash-out 10 min at 50/50 (v:v); flow rate: 6 mL/min; temperature: RT; absorbance detection at □ = 254 nm.

**Miscellaneous.** Radiosyntheses using fluorine-18, including the HPLC purifications, were performed in a 7.5-cm-lead shielded cell using a computer assisted Zymate robot system (Zymark corporation, USA).

**Fluorine-18 production.** No-carrier-added aqueous [¹⁸F]fluoride ion was produced via the [¹⁸O(p,n)¹⁸F] nuclear reaction by irradiation of a 2 mL [¹⁸O]water (> 97%-enriched) target on a CGR-MeV 520 cyclotron (17 MeV proton beam) or on a IBA Cyclone-18/9 cyclotron (18 MeV proton beam) and was transferred to the appropriate hot cell. *Target hardware* : CGR-MeV 520cyclotron : home-made, large volume, three-port, keyhole-shaped stainless steel/silver target holder (A complete description of this target hardware and -operation can be found in reference *(81)*) ; IBA Cyclone-18/9 cyclotron: commercial, large volume, two-port, stainless steel target holder equipped with a domed-end niobium cylinder insert. *Target to hot cell iiquid-transfer system* : 60 m PTFE line (0.8 mm internal diameter ; 1/16 inch external diameter), 2.0 bar He drive pressure, transfer time 3-6 min). Typical production of [¹⁸F]Fluoride at the end of bombardment for a 20 µA, 30 min (10 µA.h) irradiation: CGR-MeV 520 cyclotron: 550-650 mCi (20.3-24.0 GBq) and IBA Cyclone-18/9 cyclotron: 750-800 mCi (27.7-29.6 GBq).

**Preparation of the K[¹⁸F]F-K₂₂₂-complex.** In order to recover and recycle the [¹⁸O]water target, the 2 mL of aqueous [¹⁸ F]fluoride from the target holder were passed through an anion exchange resin (Sep-Pak^{®} Light Waters Accell^{™} Plus QMA Cartridge in the chloride form, washed with 5 mL 1 M aq. NaHCO₃ and then rinsed with 50 mL of water) by Helium pressure (1.5-2.0 bar). Helium is blown through the column to maximally extract the [¹⁸O]water. The [¹⁸F]fluoride ion is then eluted from the resin using 1.0 mL of a 4.5 mg/mL aqueous K₂CO₃ solution into a Vacutainer^{®} tube containing 12.0 to 15.0 mg of Kryptofix^{®}222 (K₂₂₂: 4,7,13,16,21,24-hexaoxa-1,10-diazabicyclo[8.8.8]hexacosane). The resulting solution was then gently concentrated to dryness at 145-150°C under a nitrogen stream for 10 min to give no-carrier-added K[¹⁸F]F-K₂₂₂ complex as a white semi-solid residue.

**Preparation of 1-[3-(2-[¹⁸F]Fluoropyridin-3-yloxy)propyl]pyrrole-2,5-dione ([¹⁸F]-1, [¹⁸F]FPyME).** 600 µL of freshly distilled DMSO containing 4.0 mg of [3-(3*-tert-*butoxycarbonylaminopropoxy)pyridin-2-yl]trimethylammonium trifluoromethanesulfonate (2) as the precursor for labeling were directly added into the Vacutainer^{®} tube containing the dried K[¹⁸F]F-K₂₂₂ complex. The tube (not sealed) was then thoroughly vortexed (15 secondes) and then placed in a heating block at 145°C for 2 min without stirring the contents. The reaction mixture was diluted with 1 mL of water and transferred on a C18 Sep-pak cartridge (PrepSep^{™} R-C18, Fisher Scientific, activated beforehand with 2 mL of EtOH and then rinsed with 10 mL of water). The tube was rinsed twice with 1 mL of water which was also transferred and added to the diluted reaction mixture on the cartridge. After addition of another 2 mL of water, the whole was passed through the cartridge. The cartridge was washed with 1 mL of water and partially dried for 0.5 min by applying a nitrogen stream. The [3-(2-[¹⁸F]fluoropyridin-3-yloxy)propyl]carbamic acid tert-butyl ester ([¹⁸F]-**3**) was eluted from the cartridge with 3 mL of CH₂Cl₂ into a 5 mL reaction vial containing 0.1 mL of TFA. Twice 1 mL of CH₂Cl₂ was used to wash the cartridge and to completely transfer [3-(2-[¹⁸F]fluoropyridin-3-yloxy)propyl]carbamic acid tert-butyl ester ([¹⁸F]-**3**) (5% of the total radioactivity amount engaged in the fluorination process was left on the cartridge). The incorporation yield was also estimated after the C18 Sep-pak cartridge elution by the CH₂Cl₂-over total eluted radioactivity (DMSO/H₂O + CH₂Cl₂) ratio. The resulting CH₂Cl₂/TFA solution (50/1, v/v) was concentrated to dryness (at 65-75°C under a gentle nitrogen stream for 4-6 min). The yield of deprotection was quantitative and no Boc-protected [¹⁰F]-**3** could be detected by radiochromatography (SiO₂-TLC, eluent : EtOAc, *R_{f}*: [¹⁸F]-**3** : 0.75 and *R_{f}* : [¹⁸ F]-**4** : 0.0). The above residue, containing 3-(2-[¹⁸F]fluoropyridin-3-yloxy)propylamine ([¹⁸F]-**4**) was redissolved in 2 mL of CH₂Cl₂ and concentrated again to dryness to minimize TFA presence (at 65-75°C under a gentle nitrogen stream for another 2-3 min). The residue, containing [¹⁸F]-**4**, was then redissolved in 0.25 mL of dioxane containing 25 mg of N-methoxycarbonylmaleimide. Aq. sat. NaHCO₃ (0.75 mL) was then added to the solution and the vessel was gently vortexed for 10 min at room temperature. The reaction mixture was diluted with 1 mL of aq. 1 M HCl and transferred onto a C18 Sep-pak cartridge (PrepSep^{™} R-C18, Fisher Scientific, activated beforehand with 2 mL of EtOH, rinsed with 10 mL of water and loaded with 5 mL of water). The tube was rinsed twice with 1 mL of water which was also transferred and added to the diluted reaction mixture on the cartridge. The whole was passed through the cartridge. The cartridge was washed with 3 mL of water and partially dried for 0.5 min by applying a nitrogen stream. The 1-[3-(2-[¹⁸F]fluoropyridin-3-yloxy)propyl]pyrrole-2,5-dione ([¹⁸F]-1, [¹⁸F]FPyME) was eluted from the cartridge with 3 mL of CH₂Cl₂. Twice 1 mL of CH₂Cl₂ was used to wash the cartridge and to completely transfer [¹⁸F]FPyME ([¹⁸F]-1) (10-25% of the radioactivity amount was left on the cartridge). The resulting CH₂Cl₂ solution was partially (not to dryness) concentrated (at 65-75°C under a gentle nitrogen stream for 2-4 min). The residue was diluted up to a volume of 1.0-1.5 mL with CH₂Cl₂ and HPLC-purified [HPLC A, *Rₜ* : 10.0-10.5 min] to give pure labeled [¹⁸F]FPyME ([¹⁸F]-**1**).

**Conjugation of TTA1 with 1-[3-(2-[¹⁸F]fluoropyridin-3-yloxy)propyl]pyrrole-2,5-dione ([¹⁸F]-1, [¹⁸F]FPyME). Preparation of [¹⁸F]c-TTA1.** To 100 µL of DMSO containing HPLC-purified [¹⁸F]FPyME ([¹⁸F]-**1**, freed from HPLC-solvents by concentration to dryness at 65-75°C under a gentle nitrogen stream) was added TTA1 (0.6-0.9 mg) dissolved in 900 µL of PBS (100 mM, pH 7.5). The reaction mixture was gently vortexed for 15 minutes and then purified by gel filtration on a NAP-10^{®} G25 Sephadex cartridge (Amersham Pharmacia Biotech). The conjugated and labeled peptide, [¹⁸F]c-TTA1, was easily separated from non-reacted [¹⁸F]FPyME ([¹⁸F]-**1**), if any, and was eluted from the cartridge in 1.5 mL of aq. 0.9% NaCl according to manufacturer's instructions.

**Formulation and Quality control of conjugated and labeled [¹⁸F]c-TTA1.** The cartridge-fraction containing the conjugated and labeled [¹⁸F]c-TTA1 was diluted with physiological saline. As demonstrated by HPLC analysis [HPLC B], radiosynthesized conjugated and labeled [¹⁸F]c-TTA1 almost co-eluted (Rt : 11.90) with an authentic sample of non-conjugated TTA1 (Rt : 11.50 min). The radiolabeled product was found to be > 95% radiochemically pure. The preparation was shown to be free of non-reacted [¹⁸F]FPyME ([¹⁸F]-**1**) and radiochemically stable for at least 240 min.

### Example 2:

### Radiochemistry

The presence of a free sulfhydryl function offers an opportunity for its labeling with [¹⁸F]FPyME (1-(3-(2-[¹⁸F]fluoropyridin-3-yloxy)propyl)pyrrole-2,5-dione), a [¹⁸F]fluoropyridine-based maleimide reagent designed for prosthetic fluorine-18-labeling of macromolecules via selective conjugation with a thiol function [1]. This fluorine-18-labeling is presented herein. [¹⁸F]FPyME was prepared using a three-step radiochemical pathway already reported [1]. Briefly, the developed procedure involves a high-yield nucleophilic *hetero*aromatic *ortho-*radiofluorination on [3-(3-tert-butoxycarbonylaminopropoxy)pyridin-2-yl]trimethyl-ammonium trifluoromethanesulfonate as the fluorine-18 incorporation-step, followed by (2) rapid and quantitative TFA-induced removal of the N-Boc-protective group and (3) optimized maleimide formation using N-methoxycarbonylmaleimide (64-77%). [¹⁸F]FPyME, after HPLC-purification (semi-preparative SiO₂ Zorbax^{®} Rx-SIL, Hewlett Packard, 9.4 x 250 mm, 5 µm), was conjugated with TTA-01 (0.650-0.850 mg) in 1 mL of a 1/9 (v/v) mixture of DMSO and 0.1 M aq. PBS (pH 7.5) at room temperature for 15 minutes. c-[¹⁸F]TTA-01 (the product of TTA-01 coupling with [¹⁸F]FPyME) was then purified by semipreparative C-18 HPLC (µBondapak^{®}, Waters, 7.8 x 300 mm, 10 µm) using a linear gradient of aq. 50 mM TEAA and ACN. Finally, desalting and formulation of c-[¹⁸F]TTA-01 in aq. 0.9% NaCl was performed using a Sephadex NAP-10 cartridge. Typically, 5.2-7.5 GBq (140-200 mCi) of radiochemically pure [¹⁸F]FPyME could be obtained after semi-preparative HPLC in 110 minutes starting from a cyclotron production batch of 37-51 GBq of [¹⁸F]fluoride (overall radiochemical yields, based on starting [¹⁸F]fluoride: 23-30% decay-corrected). Conjugation of [¹⁸F]FPyME with TTA-01 was achieved in moderate yields (50-70% non decay-corrected yield, based on radio-TLC and radio-HPLC) and c-[¹⁸F]TTA-01 was then easily purified from non-reacted [¹⁸F]FPyME using HPLC. Routinely, 0.6-1.4 GBq of HPLC-purified and formulated c-[¹⁸F]TTA-01 could be obtained in 65-75 minutes starting from the above-mentioned [¹⁸F]FPyME batch.

### Animals

Male Wistar rats (200-250 g) and athymic (*nu*/*nu*) mice (30 g Janvier) were housed separately at constant temperature (21 °C) and relative humidity (60%) under a regular light/dark schedule. Food and water were freely available. Procedures involving animals were in conformity with the CEA institutional guidelines that are in compliance with current French and European laws and poiicies.

### Tumour model

U251 cells, derived from a human glioblastoma, were cultured in RPMI 1640 (Invitrogen, France) containing 10% of foetal calf serum under 5 % of CO₂. Two millions of cells were injected subcutaneously in Matrigel (BD Bioscience, France) to adult Nu/nu mice. At time of PET examinations one month later, tumours reached approximately 500 mg.

### In vivo PET imaging

Positron emission tomography was performed as previously described [2,3]. Briefly, rats under 2% isoflurane anaesthesia were placed in the field of view of the HRRT camera (High Resolution Research Tomography, Siemens) and injected IV with 15 MBq of [¹⁸F] TTA1. For mice studies, animals were anaesthetized with isoflurane, injected on the bench with 7.4 MBq of [¹⁸F] TTA1, and placed immediately in the field of view of the microPET FOCUS 200 camera (Siemens). Dynamic sequences of whole body PET were acquired in 3D mode during 180 min after injection for rats and during 90 min after injection for mice.

### Results

### Dynamic PET imaging in rats

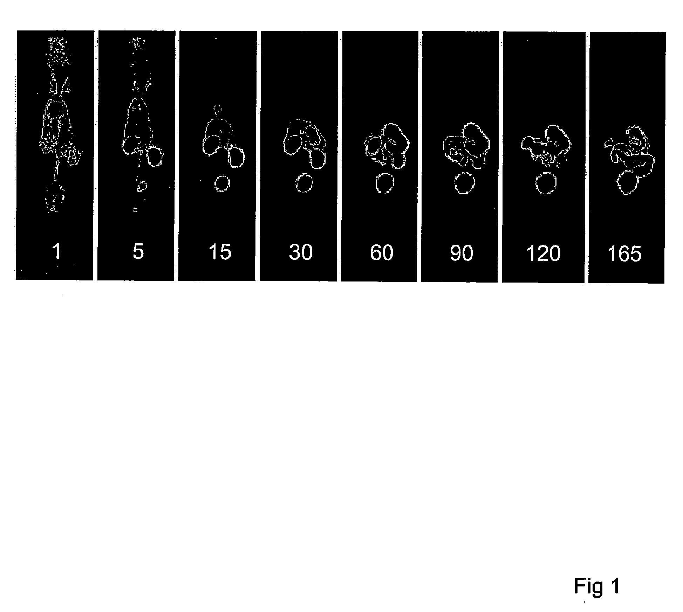

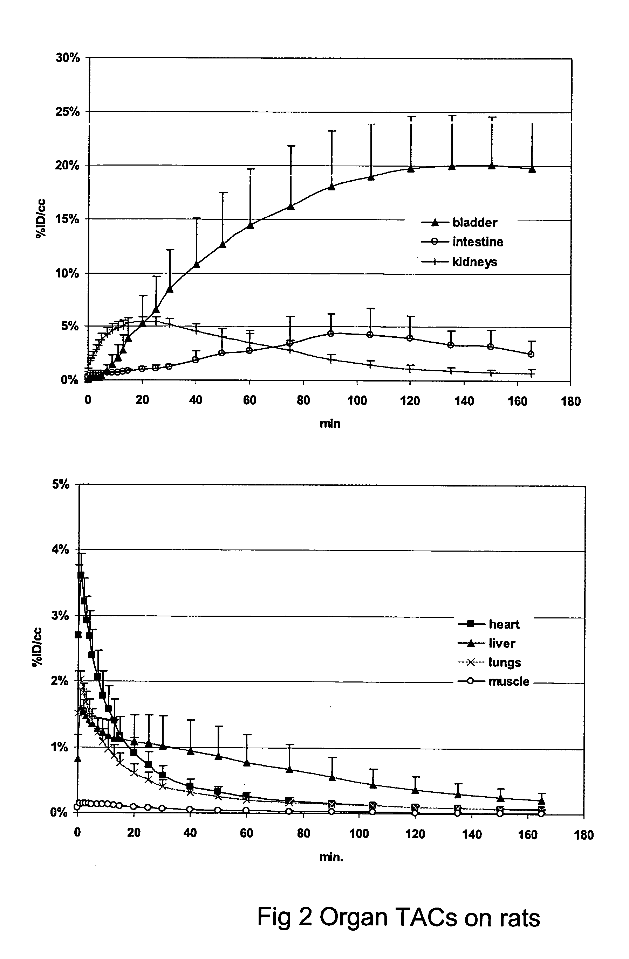

The pharmacokinetics of [¹⁸F]TTA1 was evaluated in normal rats by whole body PET imaging on the HRRT camera Fig. 1 presents whole-body projection images of a rat at different times after intravenous injection of 15 MBq of [¹⁸F]TTA1.
Organ pharmacokinetics were obtained by segmentation of PET images. Fig. 2 shows the time activity curves of [¹⁸F]TTA1 in the bladder, intestine and kidneys (upper part of the figure) and heart, liver, lungs and muscle (lower part). The large amount of radioactivity found in kidneys and bladder, and at later times, in the intestine, illustrates urinary and hepato enteric elimination pathways, respectively. In the other organs, the percentage of injected dose remained relatively low, 1.5 % of ID/cc in liver and 2 % in the lungs just after injection. Elimination of the tracer from the lungs was rapid with a similar kinetic to that observed in the heart. In the liver, the kinetics was slower than in lumps or heart, suggesting that another mechanism than vascular clearance was involved. Blood kinetics was estimated by measuring the level of radioactivity in a region of interest positioned over the heart. The maximum [¹⁸F]TTA1 concentration in the heart peaked rapidly to 3.6 +/-0.3% of ID/cc at 1 min post injection, and decreased rapidly immediately thereafter (0.07% +/- 0.01 % of ID/cc at 180 min post injection). Blood pharmacokinetic parameters were calculated from cardiac time activity curves and showed a blood clearance of 2.0 +/- 0.7 ml/min, a blood half life of 3.6 +/-0.7 min, and a T_{1/2} beta of 53 +/- 3 min (n=5).

### [¹⁸F]TTA1 for tumor imaging in mice

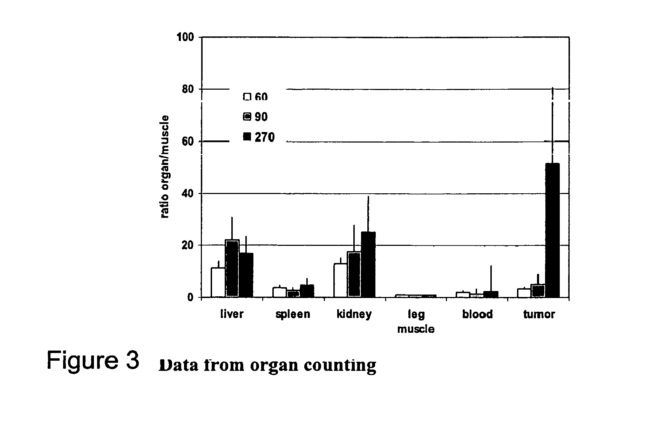

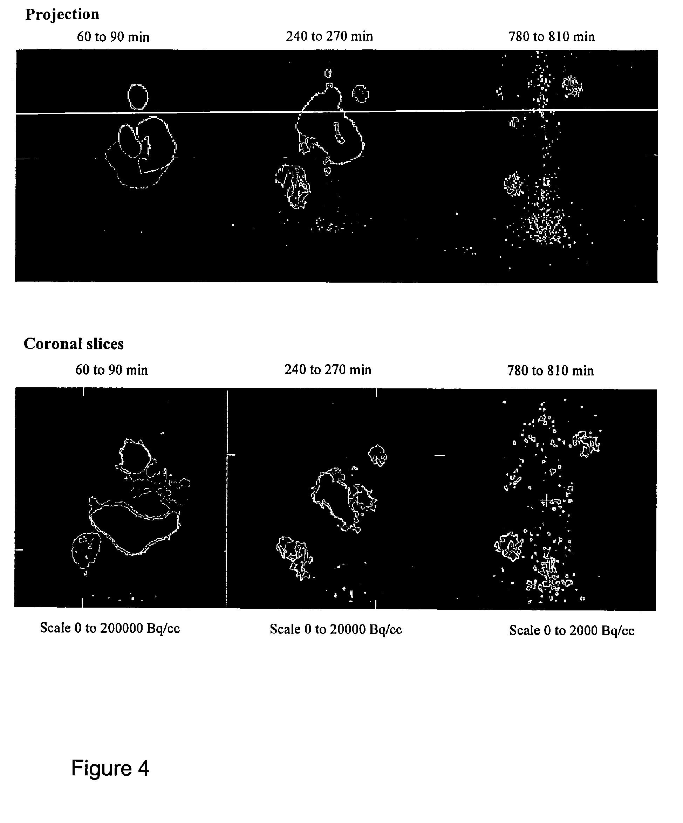

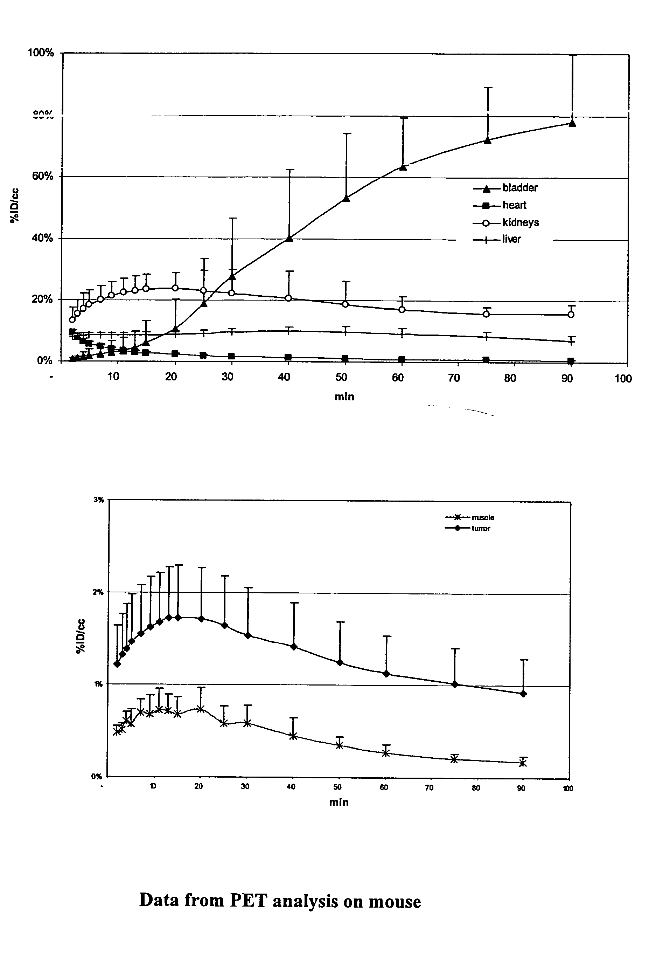

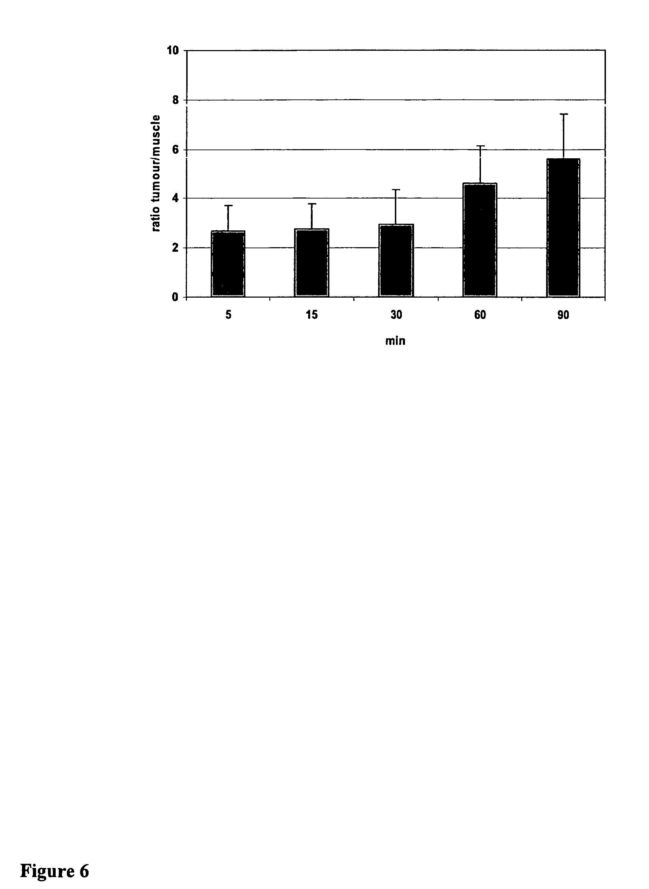

To analyse the potential of [¹⁸F]TTA1 to provide tumoral contrast *in vivo,* we used the U251 cell line to induce tumors in nude mice. These cells express high quantity of tenascin-C *in vivo* [5,6]. Tumors were obtained one month after subcutaneous administration. At that time, the mice (n= 4) were injected intravenously with [¹⁸F]TTA1 and imaged directly after injection during 90 min or, for 3 mice, up to three hours after injection during the same time. At the end of the imaging period, animals were sacrificed and organs and tumors were removed for radioactivity counting. Another group of animal were injected and sacrificed at 60 min post injection, for radioactivity counting without imaging. Figure 3 presents the ratio of radioactivity uptake in tumour over muscle at 60, 90 and 270 min after injection. During this period, the tumour to muscle ration remained relatively constant in the liver, spleen and blood illustrating the slow elimination of TTA01, and increased slightly in the kidneys.
In contest, the tumor to muscle ratio increased dramatically. At 60 min post injection the tumor to muscle ratio was 3 +/- 1; at 90 min 5+/- 3 and at 270 min 52+/- 25. The standard deviation may be explained by the heterogeneity of tumoral fixation of [¹⁸F]TTA1, as the labelled aptamer shows heterogeneous uptake in the large the tumors (figure 4). The time activity curves in the mouse organs (figure 5) were measured on PET images during the first 90 min after intravenous injection. Just after injection the level of radioactivity in the tumor corresponded to 1 % +/- 0.4 of ID/cc. This uptake increased to 1.7 +/- 0.5 % ID/cc at 20 min post injection. In comparison, uptake at the same times in the muscle was 0.4 +/- 0.1 and 0.7 +/- 0.2 % of ID/cc, respectively. Radioactivity in the muscle decreased more rapidly than in the tumor (figure 6). The tumour to muscle was stable until 30 min and then increased continually during the next 60 min, to reach 5.6 +/- 0.8 at 90 min. At this time, the radioactivity in tumors measured better on PET images, or by counting tumors was 0.9 +/-0.4 % and 1 +/- 0.2 % of ID/cc respectively. In the PET images obtained at later times, the very low radioactivity in muscle and other organs muscle can't be measured precisely. However, radioactivity in tumors was 0.3 +/- 1 % of ID/cc at 270 min post injection. The final value of the tumor to muscle ratio, quantified by counting, (figure 3) was very high 52 +/- 26 and variable: a range from 19 to 85.
As shown previously with the same aptamer [6] labelled with ^{99m}Tc, the tumor to muscle ratio increased continuously up to 17 hours. We imaged one mouse at 13 hours after injection of [¹⁸F]TTA1. For this, the mouse was injected with 140 MBq of labelled aptamer, in order to retain sufficient residual radioactivity at 13 hours (1 MB whole body expected considering the decay of F-18). At this time radioactivity had completely cleared from the digestive and urinary tracts. The only remaining hot spot corresponded to tumors and bones. Radioactivity found in bones suggests a release of 18-fluorine from labelled [¹⁸F]TTA1 which has a high affinity for skeleton. The amount of [¹⁸F]TTA1 found in tumors corresponded to 0.19 % of ID/cc in this mouse.

We found that the concentration of [¹⁸F]TTA1 was maximal in the tumor, 20 min after intravenous injection. In comparison, the unspecific uptake in muscle was maximal at 7 min after injection. Radioactivity in the muscle decreased more rapidly than that in the tumor. This difference is expressed by the enhancement of the ratio tumor to muscle starting at 2.5 one min after injection, constant until 30 min at 2.9 and increasing continuously until the end of dynamic acquisition (5.6 at 90 min). Results from PET image quantification are coherent with organ counting (ratio tumor to muscle 5.2 at 90 min post injection).

It is understood that the examples and embodiments described herein are for illustrative purpose only and that various modifications and changes in light thereof as well as combinations of features described in this application will be suggested to persons skilled in the art and are to be included within the spirit and purview of the described invention and within the scope of the appended claims. From the foregoing description, one skilled in the art can easily ascertain the essential characteristics of this invention and, without departing from the spirit and scope thereof, can make various changes and modifications of the invention to adapt it to various usages and conditions. The entire disclosure[s] of all applications, patents and publications, cited herein are incorporated by reference herein.

## Claims

1. Compound according to Formula I 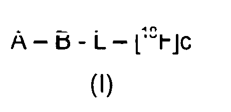 wherein
A stands for an aptamer,
B is absent or stands for a bridging structure,
L is a linker, and
[¹⁸F]c is a ¹⁸F bearing chemical entity.

2. A compound according to claim 1, wherein [¹⁸F]c is

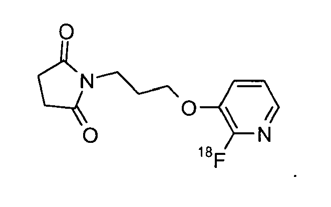

3. A compound according to claims 1 and 2, wherein A is selected from Tenascin-C binding aptamers.

4. A compound according to claims 1 to 3, wherein A is an aptamer having a binding affinity towards Tenascin-C of 1mM or less.

5. A compound according to claims 1 to 4, wherein A comprises from 10 to 50 nucleotides.

6. A compound according to claims 1 to 5, wherein B is hexyl amine.

7. A compound according to claims 1 to 6, wherein L is a chemical structure carrying at least one free mercapto-group, wherein said chemical structure comprises an amino acid sequence of 2 to 10 amino acids, a mercaptoacetyl-glycine-glycine group (MAG₂), a mercaptobutyrimidyl group, a mercapto-substituted C₁-C₄ alkyl group, a mercapto-substituted C₁-C₄ alkene group, or a mercapto-substituted C₁-C₄ aldehyde group.

8. A compound according to claims 1 to 7, wherein L is MAG₂.

9. A compound according to claims 1 to 8, wherein A is selected from the group of compounds:

TTA4:	5'-mGmGmG mAmGG mAfCmG-(CH2CH2O)6-fCGfU fCGfC fCGfU mAmAfU mGmGmA fUmGfU
fUfUfU mGfCfU fC(C6-NH2)CfC-3'-3'-T ,

TTA5:	5'-(aminomodifier C2 dT)-mA mGmGmG mAmGG mAfCmG-(CH2CH2O)6-fCGfU fCGfC fCGfU
mAmAfU mGmGmA fUmGfU fUfUfU mGfCfU fCfCfC fUmG-3'-3'-T ,

TTA7:	5'-LGLGLG LALGmG mAfCrG-(CH2OCH2)6-fCGfU fCGfC fCGfU mAmAfU mGmGmA fUmGfU
fUfUfU mGLCLU LCLCLC-(CH2)6NH2-3' , and

TTA8:	5'-mGmGmG mAmGmG mAfCrG-(CH2OCH2)6-fCGfU fCGfC fCGfU mAmAfU mGmGmA fUmGfU
fUfUfU mGmCmU mCmCmC-(CH2)6NH2-3' .

10. A compound according to claims 1 to 8, wherein A - B is selected from the group of compounds:

TTA1: 5'-NH2-C6-O-PO2-O-GGG AGG ACG-(CH2CH2O)6-CGU CGC CGU AAU GGA UGU UUU GCU
CCC UG-3'-3'-T ,

TTA2:	5'-NH2-C6-O-PO2-O-mGmGmG mAmGG mAfCmG-(CH2CH2O)6-fCGfU fCGfC fCGfU mAmAfU
mGmGmA fUmGfU fUfUfU mGfCfU fCfCfC UmG-3'-3'-T ,

TTA3: 5'-NH2-C6-O-PO2-O-mGmGmG mAmGG mAfCmG-(CH2CH2O)6-fCGfU fCGfC fCGfU mAmAfU
mGmGmA fUmGfU fUfUfU mGfCfU fCfCfC UG-3'-3'-T ,

TTA6: 5'-NH2-C6-O-SPO-O-mGmGmG mAmGG mAfCmG-(CH2CH2O)6-fCGfU fCGfC fCGfU mAmAfU
mGmGmA fUmGfU fUfUfU mGfCfU fCfCfC fUmG-3'-3'-T .

11. A composition comprising a compound according to claims 1 to 10 and a pharmaceutically acceptable carrier, diluent, excipient or adjuvant.

12. A compound according to claims 1 to 10 for use as a medicament or pharmaceutical.

13. A compound according to claims 1 to 10 for use as a diagnostic imaging agent, preferably as imaging agent for PET.

14. Use of a compound according to claims 1 to 10 for the manufacture of a medicament or pharmaceutical.

15. Use of a compound according to claims 1 to 10 for the manufacture of a diagnostic imaging agent, preferably a PET agent.

16. Use of a compound according to claims 1 to 10 for the manufacture of a diagnostic imaging agent, preferably a PET agent, for imaging tumours.
